Europäisches Patentamt

(19) European Patent Office

Office européen des brevets

(11) Publication number: **0 278 340 B1**

## (12) EUROPEAN PATENT SPECIFICATION

(45) Date of publication of patent specification: **04.08.93**

(51) Int. Cl.⁵: **C07K 15/00**, G01N 33/531, G01N 33/569, G01N 33/543

(21) Application number: **88101310.6**

(22) Date of filing: **29.01.88**

(54) **Mycoplasma membrane antigens and their use.**

(30) Priority: **05.02.87 IL 81489**
**14.08.87 IL 83546**

(43) Date of publication of application:
**17.08.88 Bulletin 88/33**

(45) Publication of the grant of the patent:
**04.08.93 Bulletin 93/31**

(84) Designated Contracting States:
**CH DE ES FR GB IT LI NL**

(56) References cited:
**EP-A- 0 196 215**
**EP-A- 0 254 384**

**BIOLOGICAL ABSTRACTS, vol. 75, no. 8, 1983, abstract no. 57153, Philadelphia, US; R. CAPPEL et al.: "Immune response to a DNA-free herpes simplex vaccine in man", & ARCH VIROL 73(1): 61-68. 1982**

(73) Proprietor: **Naot, Yehudith**
**27, Kabirim Street**
**Haifa 34 385(IL)**

Proprietor: **DIMOTECH LTD.**
**Senat House Technion City**
**Haifa 32 000(IL)**

(72) Inventor: **Naot, Yehudith**
**27, Kabirim Street**
**Haifa 34 385(IL)**

(74) Representative: **Brown, John David et al**
**FORRESTER & BOEHMERT Franz-Joseph-Strasse 38**
**W-8000 München 40 (DE)**

EP 0 278 340 B1

## Description

### Field of Invention

The invention concerns a mycoplasma membrane antigen product, a process for its preparation and use thereof for the diagnosis of mycoplasma infections.

### Background of the Invention

Mycoplasmas are the cause of many diseases such as certain types of respiratory tract inflamations, e.g. pneumonia, and certain urinary tract infections both in humans and non-human animals. Because of their slow rate of multiplication and growth, and the high costs of the growth medium needed, diagnosis of mycoplasma infections cannot be routinely effected by culturing methods. Accordingly, methods for the detection of mycoplasma infections currently used are based on detection of antibodies by immunologic assays such as, for example, complement fixation test and ELISA (Enzyme Linked Immunoabsorbent Assay), and comprise detection of antibodies to mycoplasma in body fluids.

Immunological assays for the detection of mycoplasma antibodies in the serum are based on the reaction of these antibodies with the mycoplasma antigens, a major part of which are located in the cell membrane. Hitherto, for the performance of such assays, mycoplasma cells were lysed, and the lysed cells which included the cell membrane (including all its various constituents) and components attached thereto (such as the DNA), were used as the antigen. However, even such methods, generally known by their specificity and accuracy, proved to be inaccurate and unreliable in the detection of mycoplasma induced infections, due, inter alia, to cross reactivity between mycoplasma antigens of different species and their antibodies, leading to many false positive and false negative results.

The limitations of the ELISA method for the detection of mycoplasma infections are summarized by O. Onoviran et. al. (Veterinary Record, 105: pp 165-167) in connection with mycoplasma mycoides as follows:

"ELISA is not completely specific since there was a low cross-reaction between positive contagious bovine pleuoropneumonin aera and Mycoplasma other than Mycoplasma mycoides".

ELISA is known to be a very accurate immunological assay. Accordingly, the above limitation applies a fortiori to other immunological assays such as radio-immunoassay, immunofluorescence, immunodiffusion, complement fixation, indirect hemagglutination and chemoluminescence.

### Object of the Invention

It is an object of the present invention to provide more specific mycoplasma membrane antigens, for use in immunologic methods for the diagnosis of mycoplasma infections, thereby to provide methods of diagnosis that are essentially free of false positive and false negative results for use in both human and veterinary medicine.

Further objects of the present invention will become apparent from the following description and appended claims.

### General Description of the Invention

It was found in accordance with the present invention that a fragmented mycoplasma membrane which is essentially free of DNA and RNA, can be used to advantage for diagnosis of mycoplasma infections with essentially no false positive and false negative results.

Thus, in accordance with one aspect of the present invention there is provided mycoplasma antigen material being a fragmented mycoplasma membrane material substantially free of DNA and RNA (hereinafter "mycoplasma antigen").

Surprisingly and for reasons not yet fully elucidated, an antigen-antibody reaction performed with a mycoplasma antigen according to the invention is specific with practically no cross reactions, as distinct from the cross reactions and consequential inadequate specificity of the known whole mycoplasma cells and lysed mycoplasma cells preparations.

In accordance with one embodiment of this aspect of the invention the mycoplasma antigen is in aqueous suspension. Such a suspension may be used in some immunological methods for the detection of mycoplasma antibodies such as in immunodiffusion and complement fixation.

In accordance with another embodiment of this aspect of the invention, the mycoplasma antigens are bound to a solid support such as, for example, a plate with a plurality of depressions (wells), plastic strips, beads and the like which renders them suitable for immunologic techniques such as ELISA and radio-immunoassay.

The mycoplasma antigen according to the invention may be prepared by subjecting a suspension of purified mycoplasma membranes to digestion by DNAse (deoxyribonuclease) and RNAse (ribonuclease) and to sonication.

Preferably, the DNAse and RNAse digestion precedes the sonication, the latter thus serving both for fragmentation of the mycoplasma cell membranes and the destruction of the DNAse and RNAse in the suspension.

Typically, 100-500 K units of DNAse per 1 mg of membrane protein are used in the digestion process. The preferred amount of RNAse in the digestion process was found to be 10-50 K units per 1 mg of membrane protein.

For obtaining best results care must be taken that the DNAse and RNAse used will be essentially free of proteases since these may digest the proteins of the antigen's sites of the membrane, rendering the latter less antigenic and thus less suitable for the detection of mycoplasma antibodies in body fluids.

Preferably, the sonication is intermittent and performed over several time stretches, each lasting about 40-90 seconds, e.g. three times for about 60 seconds each. It was found that a mycoplasma antigen obtained from such sonication is superior for use in the diagnosis of mycoplasma infections to an antigen obtained, for example, by three minutes continuous sonication.

The essentially DNA and RNA free mycoplasma antigen according to the invention was found to be specific in its binding to antibodies against mycoplasma, as distinct from the whole lysed mycoplasma cells used in accordance with the prior art. For diagnosis, the mycoplasma antigen according to the invention may be used in either native form, or conjugated with a suitable marker.

Mycoplasma antigens (as herein defined) conjugated with a suitable marker were found to be especially suitable for detection of mycoplasma antibodies in body fluids by various immunological methods, such as radioimmunoassay, a modified ELISA method and the like, ELISA being especially preferred.

Therefore, in accordance with a second aspect of the present invention there is provided a mycoplasma antigen-marker conjugate (hereinafter "conjugate") consisting of a fragmented membrane material essentially free of DNA and RNA bound to a suitable marker. The marker moiety may, for example, be an enzyme and such a conjugate is suitable for tests by the ELISA method.

Examples of enzyme markers to be used in accordance with this second aspect of the present invention are alkaline phosphatase, horseradish peroxidase, -galactosidase, glucose-6-phosphate dehydrogenase, glucose oxidase, lysosyme, glucoamylase and maleate dehydrogenase, which are utilized in the art in various enzyme immunoassays (Brian Wisdom Clin. Chemistry 22(8); 1243-1255, 1976). From among the above, alkaline phosphatase, horseradish peroxidase and - galactosidase are most commonly used and thus also preferred for use as enzyme label in accordance with the present invention.

In addition to enzyme markers also other markers may be used such as radioactive substances, fluorescent dyes, biotin-avidine complexes and chemiluminescence rendering the conjugate suitable for various immunological methods according to the marker employed.

A conjugate (as herein defined) according to the invention is prepared from a starting suspension comprising a mycoplasma antigen (as herein defined) and the marker to be attached thereto, and the attachment is performed by methods known in the art for the binding of markers to membrane proteins. Where, for example, an enzyme marker is being attached to the mycoplasma antigen, the conjugate may be induced to form by the addition of glutaraldehyde to the suspension. Other agents which may be employed for conjugating an enzyme to a mycoplasma antigen are, for example, periodate and S.P.D.P. [N-succinimidyl 3-(2-pyridyl dithio) propionate].

It was surprisingly found in accordance with the second aspect of the present invention that when the said marker is the enzyme alkaline phosphatase, the highest activity of conjugate is obtained from a starting suspension comprising about 800 - 1200 Units [Unit defined as the amount of enzyme that will hydrolise 1.0 $\mu$mole of p-nitrophenyl phosphate per minute at a pH of 10.4 (adjusted with glycine buffer) at 37°C] of enzyme for 1 mg of proteins in the mycoplasma antigen.

By a third aspect, the invention provides an in vitro method of diagnosing mycoplasma infections by the detection of antibodies against mycoplasma in a body fluid by means of mycoplasma antigen or conjugate (both as herein defined).

The method constituting this third aspect of the invention is applicable in either human or veterinary medicine.

By way of example, a body fluid sample to be tested, which, if desired, may be diluted, is incubated with a matrix such as a micro-ELISA well which has been precoated with at least one kind of anti-isotype antibodies selected from the group of anti-IgM, anti-IgG, anti-IgA, anti-IgD and anti-IgE, for an amount of time sufficient for the binding of immunoglobulin isotypes present in the body fluid sample to the anti-isotype antibodies bound to the matrix. After removing excess, unbound fluid sample components, the matrix is incubated with a suspension of a mycoplasma antigen-marker conjugate and the amount of said marker conjugate on the matrix is determined.

The presence and level of antibodies against a specific mycoplasma species in said body fluid is determined by comparing the amount of conjugate on the matrix obtained from the tested body fluid, with the amount obtained by a similar test in which control samples (control positive and control negative) of anti-mycoplasma antibodies are used and in this way a mycoplasma infection is diagnosed.

If the marker moiety of the conjugate is an enzyme, such as alkaline phosphatase, the determination of the conjugate on the matrix will comprise the step of adding a substrate for the conjugated enzyme to the incubated mixture and after an adequate incubation time the concentration of such substrate or of a degradation product thereof is determined. In the case of alkaline phosphatase, for example, it is possible to determine optically the level of the degradation product by measuring the absorbance of the solution.

Diagnosis on the basis of other markers, such as fluorescent dyes, radioactive substances, biotin-avidine complexes and chemiluminescence, may be performed by conventional methods known per se.

By a fourth aspect, the invention also provides a kit for carrying out the in vitro method for diagnosis of mycoplasma infection according to the invention, which kit comprises:

(a) a matrix carrying one or more types of anti-isotype antibodies selected from the group consisting of anti-IgM, anti-IgG, anti-IgE, anti-IgD and anti-IgA;

(b) an amount of conjugate (as herein defined);

and, where the marker moiety of said conjugate is an enzyme

(c) a substrate for an enzymatic reaction.

The kit according to the invention may also comprise one or more standard control sera (control positive and/or control negative), to which the results obtained from a test serum may be compared.

The kits according to the invention are suitable for use in human and veterinary medicine.

The mycoplasma antigens of the present invention fulfill a long felt need in that they enable for the first time to diagnose mycoplasma infections substantially without false positive and false negative results.

## Description of a Specific Embodiment

In the following, a detailed description is given with reference to a specific embodiment of the various aspects of the present invention namely obtaining purified mycoplasma pneumoniae (hereinafter "M. pneumoniae") membrane fragments; preparing a conjugate of purified M. pneumoniae antigen with the enzyme alkaline phosphatase; and utilization of the conjugate for the diagnosis of an M. pneumoniae infection. It should be understood that this is by way of example only and the invention is not limited thereto.

Generally, the performance of the invention comprises the following three steps:

## Step A - Preparation of essentially DNA and RNA free fragmented M. pneumoniae membranes

The M. pneumoniae cells are cultivated, harvested and lysed as known in the art [Tully, et al., Science 195: pp. 892-894, (1977); Razin, S., in: Methods in Mycoplasmology, S. Razin and J. G. Tully (eds.) Academic Press New York (1983), Vol. 1, pp. 225-233; Rottem, S., in: Methods in Mycoplasmology, S. Razin and J. G. Tully (eds.) Academic Press New York (1983), Vol. 1, pp. 221-223; Razin, S. and Rottem, in: Biochemical analysis of membranes, Maddy, A.H. (ed.), Chapman and Hall, London (1976) pp. 3-26]. Lysis is preferably performed by treatment with non-ionic surfactant such as, for example, digitonin or with glycerol followed by osmotic lysis, e.g. with prewarmed distilled water. The lysed M. pneumoniae cell suspension is centrifuged and the sediment (consisting essentially of purified M. pneumoniae membranes) is resuspended in a solution of a phosphate buffered saline (PBS) at a pH of about 7.

A PBS solution of DNAse and RNAse essentially free of protease, containing also $MgCl_2$, is admixed to the suspension. Upon suitable incubation, this step essentially eliminates mycoplasmal ribonucleic- and deoxyribonucleic-acids and their complexes from the mycoplasma membranes. The optimal concentration of DNAse and RNAse was found to be within the ranges of 100-500 K units and 10-50 K units per mg of membrane protein, respectively.

The enzymatic reaction is stopped by the addition of cold PBS and the suspension is centrifuged, the supernatant is removed and the sediment obtained is resuspended in PBS and thereafter sonicated. The sonication is preferably performed for several short periods of about 40 to 90 seconds each, e.g. 3 times at 60 sec. Preferably after the suspension is cooled during sonication, e.g. by means of an ice bath.

The resulting product is used for further processing.

Step B - Conjugation of DNA and RNA free fragmented M. pneumoniae membrane with the enzyme alkaline phosphatase

Alkaline phosphatase is stored in an aqueous solution of ammonium sulfate. In order to obtain pure alkaline phosphatase, the ammonium sulfate has to be removed. This is preferably achieved by centrifugation of the alkaline phosphatase solution, removal of the supernatant and an optional additional centrifugation, by which a sediment of pure alkaline phosphatase is obtained.

The suspension obtained by Step A above is admixed to the alkaline phosphatase sediment, the amount of the suspension being such as to insure that about 800-1200 units of alkaline phosphatase per 1 mg of mycoplasma membrane proteins will be present in the resulting suspension, which is then dialysed, preferably in the cold (2-8°C), against large volumes of PBS. After dialysis glutaraldehyde is added to a final concentration of 0.1-0.3% (w/v), preferably about 0.2% (w/v). The suspension is then incubated at room temperature, at a pH within the range of about 6.5 to 7.5, preferably 7.2-7.4. The resulting conjugate suspension is dialysed against large volumes of PBS, preferably at low temperatures (2-8°C).

Following the dialysis, the suspension is stored in a solution containing tris buffer, magnesium chloride (serving as coenzyme), sodium azide (serving as preservative) and bovine serum albumin (serving as suspension stabalizer as an enzyme activity preserver).

Step C - Use of the conjugate of Step B in an ELISA test for determination of antibodies against M. pneumoniae in a serum

A modified ELISA methodology was employed. The ordinary ELISA method is described by Engvall and Perlmann (Immunochemistry 8: 871-874, (1971)) and by Van Weemen and Schuurs (F.E.B.S. Lett. 15: 232-236, (1971)).

A suitable carrier, e.g. a 96 well ELISA plate, is coated as known per se with anti-IgM, anti-IgG, anti-IgA, anti-IgD and/or anti-IgE. After washing, the wells are post coated, e.g. with bovine serum albumin or fetal calf serum or any other suitable material, and washed again.

Samples of the serum to be tested, diluted if desired, are contacted with a coated carrier. In order to increase the accuracy and reliability of the results, it may be advantageous to run several parallel tests for each serum. Each sample is incubated with a coated carrier and upon completion of the reaction each carrier is washed to remove unadsorbed serum components.

A suspension of conjugate obtained in Step B above is added onto each carrier which is then incubated. Conjugate is bound to the carrier and upon completion excess conjugate is washed off and an aqueous solution of a substrate for alkaline phosphatase, e.g. p-nitrophenyl phosphate, is contacted with the carrier and the carrier with the solution is incubated for an additional time, e.g. 60 minutes, at 37°C. Thereafter, the optical absorbance of the enzymatic reaction product, e.g. p-nitrophenyl, is determined using a spectrophotometer generating light at 405 nm. In the event the carrier is an ELISA well plate, a micro-ELISA spectrophotometer may be employed.

Parallel control tests (positive, negative and mid-range positive) may be performed, if desired, and the results obtained from the tested serum are compared thereto.

The invention will now be further specifically described in the following, non-limiting Examples and Tests:

Example 1:

Preparation of fragmented, DNA and RNA free M. pneumoniae antigen

M. pneumoniae was cultivated in a manner similar to the one described by Tully et. al. (1977 Science 195: p. 892-4). The mycoplasma cells were harvested by centrifugation at 14,000 g for 50 minutes in a temperature of 4°C and thereafter washed three times with a solution of 0.25 M of sodium chloride and finally, the resulting cell suspension was diluted 100 fold with 0.25 M of sodium chloride solution.

The suspension was incubated with digitonin (25 ug/ml) for 30 minutes at 37°C, followed by the addition of 10 volumes of prewarmed, double distilled water and further incubation for 30 minutes at 37°C. The suspension of lysed cells thus obtained was centrifuged at 45,000 g for 90 minutes and the resulting sediment was resuspended in 0.1 M PBS at a pH of 7.0. The suspension of mycoplasma membranes was kept at -20°C until further use.

A solution of PBS (0.1 M at pH of 7.0) containing DNAse (type IV, manufactured by SIGMA, catalog No. R-5025) and RNAse (type I, manufactured by SIGMA, catalog No. R-5503) which are essentially protease free, and magnesium chloride (to a final concentration of 0.02 M), was admixed with the above M. pneumoniae membrane suspension. The amount of DNAse and RNAse added was 200 K units and 20 K units per mg of M. pneumoniae membrane protein, respectively. The enzymatic reaction was stopped by the addition of an additional volume of cold PBS and this was followed by centrifugation at 45,000 g for about 90 minutes in cold.

The sediment was resuspended in PBS, the volume of the PBS being such as to insure a final protein concentration of 5-20 mg/ml. The suspension was sonicated 3 times at 12 mA, low magnitude, each time for 60 seconds, and throughout the sonication the suspension was kept in an ice bath.

A suspension of purified M. pneumoniae FH membrane fragments essentially free of DNA and RNA was obtained.

Example 2:

Conjugation of the M. pneumoniae membrane antigens with the enzyme alkaline phosphatase

Alkaline phosphatase is commerically available, dissolved in a 3.2 M ammonium sulfate (SIGMA, type VII, catalog No. P-5521 or P-5526, having activity of about 1000 U/mg). In order to remove the ammonium sulfate, the solution is centrifuged in 4°C at 1,000 g for about 15 minutes, the supernatant is removed and the sediment is subjected to an additional centrifugation, as above.

The alkaline phosphatase pellet obtained from the centrifugation was mixed with the M. pneumoniae membrane fragments suspension, the mixture suspension containing about equal amounts (by weight) of alkaline phosphatase and M. pneumoniae membrane proteins. The mixture suspension was transferred to a dialysis tube and dialysis was carried out overnight at a temperature of 2-8°C against 500-1,000 volume of PBS. The dialysis was followed by the addition of glutaraldehyde to a final concentration of 0.2% w/v and incubation for two hours at room temperature. PBS was added into the resulting suspension which was then dialysed again at a temperature of 2-8°C overnight against 500-1,000 volumes of PBS.

Following the final dialysis, the suspension was transferred into a tris buffer solution at pH 8 containing 0.05 M tris, 0.001 M magnesium chloride, 0.02% w/v of sodium azide and 5% w/v of bovine serum albumin.

A suspension of conjugate was thus obtained.

Example 3:

Preparation of DNA and RNA free fragmented mycoplasma membranes from other mycoplasma species

In similar manner as in Example 1, fragmented membrane products essentially devoid of DNA and RNA were prepared from the following mycoplasma species:

M. pneumoniae PI 1428, M. hominis, M. fermentans, Ureaplasma urealyticum, M. orale, M. pulmonis, M. salivarium and Acholeoplasma laidlawii.

Example 4:

Conjugation of M. pulmonis membrane antigens with the enzyme alkaline phosphatase

In a similar manner as in Example 2, conjugates of M. pulmonis antigens of Example 3 with the enzyme alkaline phosphatase were prepared.

Example 5:

Use of the conjugate of Example 2 for the detection of specific antibodies in serum by the ELISA method

A 96 well ELISA plate was coated with anti-IgM antibodies and post-coated with bovine serum albumin by methods known per se (Naot Y. and Remington J. S., J. Infect. Dis. 142: 757-766, 1980).

Serum was tested for antibodies against M. pneumoniae in the following manner:

(a) Three 100 μl samples of serum or of PBS diluted serum were added into three pre-coated wells and incubated for 1 h at 37°C.

(b) Excess serum was removed and the wells were washed three times with a pH 7.4 PBS solution also containing 0.05% of TWEEN 20 with a 5 minute incubation period between successive washings.

(c) A suspension of the conjugate of Example 2 was added into the wells and incubated for 1 h at 37°C.

(d) Excess conjugate was washed off by a procedure similar to that of step (b).

(e) A solution containing 1 mg/ml p-nitrophenyl phosphate, 1 mM of $MgCl_2$ and 0.05 M of a carbonate buffer and having a pH of 9.8, was added into the wells and incubated therein for an additional 1 h at 37°C.

(f) The optical density of the enzymatic reaction product was determined in the wells using spectrophotometers with a light wavelength of 405 nm.

For comparison, antibodies against M. pneumoniae were tested in a similar manner with conjugates (in step (c)) not according to the invention.

Results obtained by this method are demonstrated in the Tests below:

Test No. 1:

Comparison of the specificity of an alkaline phosphatase conjugate prepared from non-sonicated, membranes of M. pneumoniae cells with a similar conjugate from sonicated membranes of M. pneumoniae

Control positive and control negative sera of different rates of dilution were tested in accordance with the method of Example 5, the suspension added in stage (c) thereof comprising either non-sonicated or sonicated membranes of M. pneumoniae alkaline phosphatase conjugate. Each of the suspensions comprised membrane proteins at a concentration of 10 ug/ml.

In each case, the optical density of the positive (P) control serum and the negative (N) control serum was measured and the ratio P/N was calculated.

The results obtained are shown in the following Table 1 and demonstrate that fragmentation by sonication significantly increases the sensitivity of the testing method.

TABLE 1

| Alkaline phosphatase conjugated to: | Serum Type | ABSORBANCE at a serum dilution of: | | | | | | | |
|---|---|---|---|---|---|---|---|---|---|
| | | 1:20 | 1:80 | 1:320 | 1:1280 | 1:5120 | 1:20480 | 1:81920 | 1:327680 |
| M. pneumoniae membranes | Positive serum | 0.473 | 0.364 | 0.355 | 0.265 | 0:238 | 0.190 | 0.192 | 0.175 |
| | Negative serum | 0.231 | 0.176 | 0.174 | 0.165 | 0.156 | 0.149 | 0.170 | 0.161 |
| | P/N | 2.04 | 2.06 | 2.04 | 1.6 | 1.52 | 1.27 | 1.12 | 1.08 |
| M. pneumoniae sonicated membranes | Positive serum | 0.798 | 0.582 | 0.498 | 0.489 | 0.353 | 0.270 | 0.164 | 0.164 |
| | Negative serum | 0.200 | 0.142 | 0.123 | 0.128 | 0.114 | 0.111 | 0.099 | 0.098 |
| | P/N | 3.99 | 4.09 | 4.04 | 3.82 | 3.09 | 2.43 | 1.65 | 1.67 |

Test No. 2:

Comparison of the specificity of alkaline phosphatase conjugate prepared from sonicated membranes of M. pneumoniae with a similar conjugate of DNA and RNA free, sonicated M. pneumoniae membrane according to the invention (mycoplasma antigen)

Control positive and control negative sera of different rates of dilution were tested in accordance with the method of Example 5, the suspension added in stage (c) thereof comprising either alkaline phosphate conjugates of sonicated membranes of M. pneumoniae or of DNA and RNA free sonicated membranes of M. pneumoniae according to the invention.

Different concentrations of the conjugates were used and the results of the optical density measured in each case, shown in the following Table 2, demonstrate the remarkable increase in sensitivity of the method according to the invention over the similar method using conjugates obtained from sonicated membranes.

TABLE 2

| Alkaline Phosphatase conjugated to: | Conjugate-protein concentration | Sera | ABSORBANCE at a serum dilution of: | | | | | | | |
|---|---|---|---|---|---|---|---|---|---|---|
| | | | 1:50 | 1:100 | 1:200 | 1:400 | 1:800 | 1:1600 | 1:3200 | 1:6400 |
| M. pneumoniae Antigens sonicated | 10µg/ml | Positive | 0.614 | 0.552 | 0.506 | 0.566 | 0.465 | 0.446 | 0.489 | 0:448 |
| | | Negative | 0.240 | 0.256 | 0.136 | 0.144 | 0.133 | 0.130 | 0.145 | 0.132 |
| | 5µg/ml | Positive | 0.308 | 0.243 | 0.252 | 0.222 | 0.235 | 0.244 | 0.215 | 0.240 |
| | | Negative | 0.154 | 0.148 | 0.117 | 0.158 | 0.121 | 0.106 | 0.115 | 0.100 |
| | 2.5µg/ml | Positive | 0.213 | 0.175 | 0.157 | 0.170 | 0.156 | 0.156 | 0.153 | 0.151 |
| | | Negative | 0.073 | 0.086 | 0.081 | 0.074 | 0.072 | 0.080 | 0.074 | 0.072 |
| M. pneumoniae Antigens digested with DNAse & RNAse and sonicated | 10µg/ml | Positive | 1.376 | 1.270 | 1.171 | 1.014 | 1.056 | 1.060 | 1.056 | 1.060 |
| | | Negative | 0.182 | 0.153 | 0.141 | 0.125 | 0.120 | 0.141 | 0.116 | 0.112 |
| | 5µg/ml | Positive | 0.811 | 0.731 | 0.702 | 0.684 | 0.662 | 0.675 | 0.622 | 0.675 |
| | | Negative | 0.113 | 0.087 | 0.101 | 0.085 | 0.081 | 0.070 | 0.084 | 0.075 |
| | 2.5µg/gl | Positive | 0.482 | 0.384 | 0.362 | 0.362 | 0.366 | 0.339 | 0.338 | 0.375 |
| | | Negative | 0.073 | 0.065 | 0.060 | 0.050 | 0.044 | 0.045 | 0.055 | 0.061 |

EP 0 278 340 B1

Test No. 3:

Comparison of the specificity of alkaline phosphatase conjugates according to the present invention obtained at various weight ratios of M. pneumoniae membrane proteins to alkaline phosphatase.

Conjugation of sonicated, substantially DNA and RNA free membranes of M. pneumoniae with alkaline phosphatase was performed as described in a suspension wherein the weight ratios of membrane protein:alkaline phosphatase (SIGMA type VII) were 4:1, 2:1 and 1:1.

Comparisons of the performance of these three different conjugates were made by the method in accordance with Example 5, using as control a positive control serum, a low positive control serum and a negative control serum, all being diluted 50 fold over the original concentration. Different concentrations of conjugates were used and in each case the optical density was measured and the ratio P/N was calculated.

The results shown in the following Table 3 demonstrate that the highest sensitivity was obtained when using conjugates prepared from membrane proteins:alkaline phosphatase ratio of 1:1. Since the activity of the alkaline phosphatase is about 1000 U/mg, it may be concluded that the best ratio of enzyme to membrane proteins is 1000 U enzyme:1 mg membrane protein.

TABLE 3

| Conjugate prepared at a ratio of mycoplasma antigen proteins to enzyme of: | M. pneumoniae antigen proteins concentration | Negative serum | | Positive serum | | Low positive serum | |
|---|---|---|---|---|---|---|---|
| | | Mean OD | P/N | Mean OD | P/N | Mean OD | P/N |
| 4:1 | 5 µg/ml | 0.104 | 1 | 0.590 | 5.67 | 0.341 | 3.28 |
| | 2.5 µg/ml | 0.087 | 1 | 0.256 | 2.9 | 0.174 | 2.0 |
| | 1.66µg/ml | 0.075 | 1 | 0.240 | 3.2 | 0.147 | 1.96 |
| | 1.25µg/ml | 0.086 | 1 | 0.204 | 2.37 | 0.146 | 1.69 |
| | 1 µg/ml | 0.071 | 1 | 0.178 | 2.5 | 0.120 | 1.69 |
| 2:1 | 5 µg/ml | 0.189 | 1 | 1.469 | 7.77 | 0.741 | 3.9 |
| | 2.5 µg/ml | 0.133 | 1 | 0.788 | 5.9 | 0.392 | 2.9 |
| | 1.66µg/ml | 0.105 | 1 | 0.562 | 5.3 | 0.293 | 2.8 |
| | 1.25µg/ml | 0.095 | 1 | 0.372 | 3.9 | 0.254 | 2.67 |
| 1:1 | 5 µg/ml | 0.331 | 1 | -- | --- | 1.506 | 4.5 |
| | 2.5 µg/ml | 0.206 | 1 | 1.488 | 7.2 | 0.838 | 4.07 |
| | 1.66µg/ml | 0.164 | 1 | 1.060 | 6.46 | 0.613 | 3.7 |
| | 1.25µg/ml | 0.135 | 1 | 0.863 | 6.4 | 0.450 | 3.3 |
| | 1 µg/ml | 0.132 | 1 | 0.737 | 5.6 | 0.396 | 3.0 |

EP 0 278 340 B1

Test No. 4:

Detection of antibodies against M. pulmonis in a rat serum.

A 96 well ELISA plate was coated with rabbit antibodies to rat immunoglobulins and then post coated with bovine serum albumin in a similar manner as Example 5.

Rat positive and negative sera diluted at various levels were added to the wells and the detection of antibodies was performed in a similar manner to Example 5 wherein in stage (c) the conjugates of Example 4 were used.

The results shown in Table 4 demonstrate that negative sera exhibited results similar to PBS, and that antibodies against M. pulmonis can be clearly detected even at high degrees of serum dilution.

## TABLE 4

ABSORBANCE   405nm

at a Serum Dilution of:

|  | 1:20 | 1:80 | 1:320 | 1:1280 | 1:5120 | 1:20480 | 1:81920 | 1:327680 |
|---|---|---|---|---|---|---|---|---|
| Positive serum | 0.867 | 0.543 | 0.520 | 0.286 | 0.260 | 0.183 | 0.151 | 0.140 |
| Negative serum | 0.133 | 0.126 | 0.127 | 0.133 | 0.132 | 0.140 | 0.135 | 0.133 |
| PBS | 0.137 | 0.123 | 0.120 | 0.125 | 0.137 | 0.140 | 0.124 | 0.130 |

Test No. 5:

Specificity of the method of Example 4 in clinical testing for specific M. pneumoniae antibodies in sera.

Mycoplasma antigens and conjugates were prepared as described in Examples 1 and 2. The following sera were tested for M. pneumoniae antibodies in accordance with the method of Example 5:

(a) 114 serum samples obtained from 114 healthy volunteers;

(b) 238 serum samples obtained from 204 patients suffering from bacterial or viral pneumonia;

(c) 60 serum samples obtained from 60 patients having high titers of rheumatoid factor or anti-nuclear antibodies or both.

All sera were found to be negative for M. pneumoniae specific antibodies by the IgM ELISA method of Example 5, demonstrating the specificity of the method according to the invention.

**Description of the Drawings**

In the following Tests 5 and 6, reference is made to the annexed drawings in which:

Fig. 1 is a graphical representation showing results of a typical experiment to demonstrate the specificity of mycoplasma antigens according to the invention;

Fig. 2 shows electrophoresis pattern runs of M. pneumoniae antigens, according to the invention, together with other M. penumoniae antigens and molecular weight standards.

Test No. 6:

Specificity of M. pneumoniae conjugates prepared in accordance with the invention.

The specificity of the method of diagnosis according to the present invention was tested. To this end control positive and control negative sera were tested in accordance with the method of Example 5. Several series of tests were conducted. In one series, M. pneumoniae FH conjugate was added to the microwells in stage (c) together with various concentrations of non labeled native M. pneumoniae FH antigen. In another series, M. pneumoniae FH conjugate was added to the microwells in stage (c) together with various concentrations of non labeled native Acholeoplasma laidlawii antigens. In yet another series, M. pneumoniae conjugate was added to the microwells in stage (c) together with PBS.

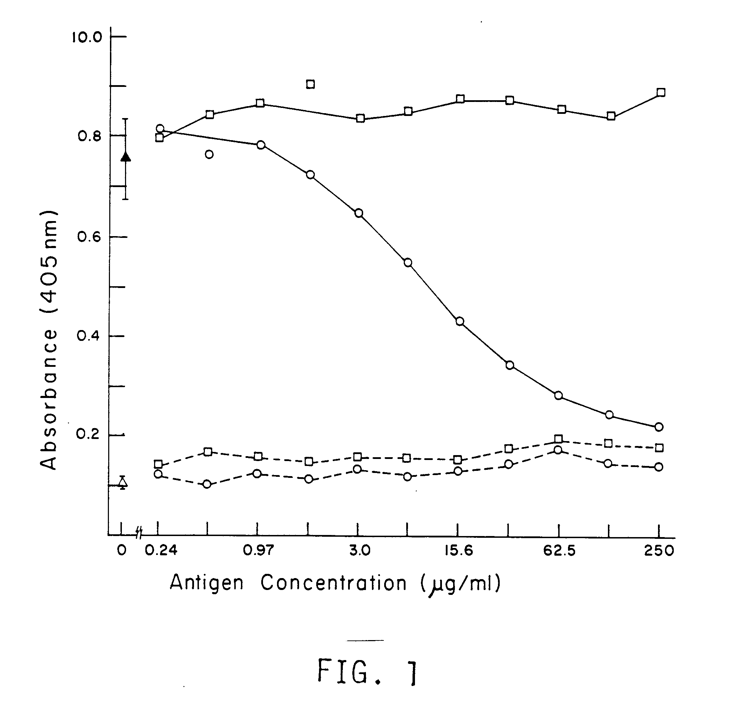

The results of these competition reactions are shown in Fig. 1 in which the optical absorbance obtained from either control positive (full lines) or control negative (broken lines), is plotted as a function of the mycoplasma antigen concentration. The results obtained with M. pneumoniae antigens are represented by empty circles (O); those obtained with Acholeoplasma laidlawii are represented by empty squares (□); those obtained with PBS are represented by triangles, either empty (△) for results with control positive sera, or filled (▲) for results with control negative sera (the bars show the standard deviation of the mean). The inhibitory effect on binding of the conjugate to the antibodies is evident from the decrease in absorbance, and occurred only with non labeled M. pneumoniae antigens.

A similar inhibition of binding was also observed when non labeled M. pneumoniae PI 1428 antigens of Example 3 were added to the microwells instead of the non labeled M. pneumoniae antigens. All other antigens of Example 3 behaved like Acholeoplasma laidlawii antigens.

This result demonstrates the specificity of the method according to the present invention.

Test No.7:

Characterization of DNA and RNA free, sonicated M. pneumoniae antigens of the invention on SDS-polyacrylamide gel electrophoresis (SDS-PAGE) and comparison to other M. pneumoniae antigens

Samples to be tested were dissolved in Sample Buffer (consisting of 1% SDS, 7mM 2- -mercapthoethanol, 10% glycerol and 50 mM Tris-HI buffer at a pH of 6.8), heated to 100°C for 4 minutes and layered on gel together with molecular weight standards. SDS-PAGE was performed on 7.5% separating slab gels and 4% stacking gel as known in the art (c.f. Laemmli U. K., 1970, Nature, London 227: 680; Weber, K. and Osborn, M., 1969, J. Biol. Chem. 244: 4406). Electrophoresis was carried out under a constant 12 uA current for 16 hours.

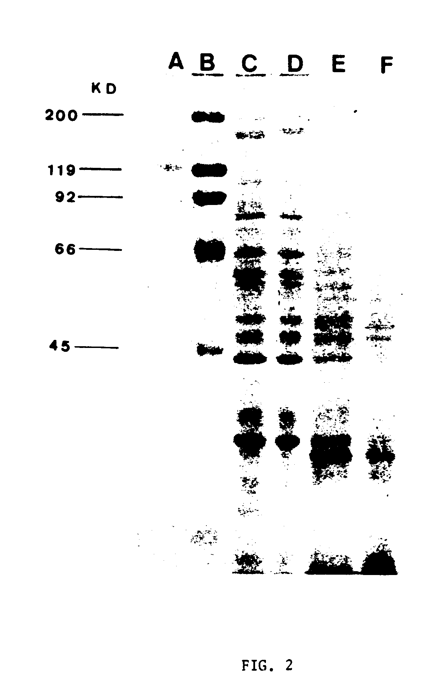

The results are shown in Fig. 2 in which:

A and B are runs of molecular weight standards;

C is a run of a sample of M. pneumoniae membranes;

D is a run of a sample of sonicated M. penumoniae membranes;

E is a run with DNAse and RNAse digested M. pneumoniae membranes;

F is a run with sonicated DNAse and RNAse digested M. pneumoniae membranes prepared in accordance with Example 1.

The molecular weight of the proteins in the M. pneumoniae antigens according to the invention (F) was thus found to be within the range of 10,000 to 60,000 daltons.

When compared to other M. pneumoniae antigens, not according to the invention, it is apparent that the M. pneumoniae antigen according to the invention consists of fewer types of proteins.

**Claims**

1. A fragmented mycoplasma membrane material referred to herein as mycoplasma antigen characterized in being essentially free of DNA and RNA.

2. A mycoplasma antigen according to claim 1 suspended in an aqueous solution.

3. A mycoplasma antigen according to claim 1 bound to a solid support.

4. A mycoplasma antigen according to claim 1 characterized in that the mycoplasma is selected from the group of M. pneumoniae FH, M. pneumoniae PI 1428, M. hominis, M. fermentans, Ureaplasma urealyticum, M. orale, M. pulmonis, M. salivarium and Acholeoplasma laidlawii.

5. An M. pneumonia antigen according to claim 4 characterized in that the proteins thereof have a molecular weight in the range of about 10,000 to about 60,000 Daltons.

6. A process for the preparation of a mycoplasma antigen according to any one of claims 1 to 5, characterized by the steps of lysing a mycoplasma and subjecting the membrane thereof to DNAse and RNAse digestion and sonication.

7. A process according to claim 6 characterized in that said digestion precedes the sonication.

8. A process according to claim 6 or 7 characterized in that the amount of DNAse is from about 100 to about 500 K units per 1 mg of mycoplasma membrane protein.

9. A process according to any one of claims 6 to 8 characterized in that the amount of RNAse is from about 10 to about 50 K units per 1 mg of mycoplasma membrane protein.

10. A process according to any one of claims 6 to 9 characterized in that the sonication is intermittent with each individual sonication period lasting for about 40 to about 90 seconds.

11. A mycoplasma antigen according to any one of claims 1 to 5, characterized in that it is prepared by the process of any one of claims 6 to 10.

12. A conjugate characterized by a mycoplasma antigen according to any one of claims 1 to 5 and 11 bound to a marker.

13. A conjugate according to claim 12 characterized in that the marker moiety is an enzyme.

14. A conjugate according to claim 13 characterized in that the enzyme is selected from the group of alkaline phosphatase, horseradish peroxidase, -galactosidase, glucose-6-phosphate dehydrogenase, glucose oxidase, lysosyme, glucoamylase and maleate dehydrogenase.

15. A conjugate according to claim 11 characterized in that the marker is selected from the group of radioactive substances capable of conjugating with mycoplasma antigen, fluorescent dyes, biotin-avidine complexes and chemiluminescent substances all of which are conventionally used as markers.

**16.** An alkaline phosphatase conjugate according to claim 14 characterized in that it is prepared from a starting suspension comprising about 800-1200 Units of alkaline phosphatase to 1 mg of mycoplasma membrane proteins.

**17.** An in vitro method for the diagnosis of mycoplasma infections in a human or non-human animal comprising in vitro detection of antibodies against mycoplasma in a body fluid, characterized by using a mycoplasma antigen according to any one of claims 1 to 5 and 11.

**18.** An in vitro method for the diagnosis of mycoplasma infections in a human or non-human animal comprising in vitro detection of antibodies against mycoplasma in a body fluid, characterized by using a mycoplasma antigen conjugate according to any one of claims 12 to 16.

**19.** A method according to claim 18, characterized by:
(a) incubating a native or diluted body fluid sample with a matrix bearing at least one kind of anti-isotype antibodies selected from the group of anti-IgM, anti-IgG, anti-IgA, anti-IgD and anti-IgE;
(b) removing the fluid sample from the matrix and washing the matrix;
(c) incubating the matrix with an aqueous suspension of a conjugate according to any one of claims 13 to 18; and
(d) determining the amount of said conjugate on the matrix.

**20.** A method according to claim 19 characterized by using a conjugate in which the marker moiety is an enzyme and the determination of the amount of said conjugate on the matrix comprises:
(a) incubation with an aqueous substrate medium for the enzyme in the conjugate;
(b) determination of the concentration of the substrate or a degradation product thereof.

**21.** For the performance of the method of claims 19 or 20, a kit characterized by:
(a) a matrix bearing at least one type of anti-isotype antibodies selected from the group consisting of anti-IgM, anti-IgG, anti-IgE, anti-IgD and anti-IgA; and
(b) an amount of a conjugate according to any one of claims 12 to 16.

**22.** A kit according to claim 21, in which said conjugate has an enzyme marker moiety, characterized by including a substrate for an enzymatic reaction.

**Patentansprüche**

**1.** Fragmentiertes Mycoplasma-Membranmaterial, das hier als Mycoplasma-Antigen bezeichnet wird, dadurch gekennzeichnet, daß es im wesentlichen frei von DNA und RNA ist.

**2.** Mycoplasma-Antigen nach Anspruch 1, welches in einer wäßrigen Lösung suspendiert ist.

**3.** Mycoplasma-Antigen nach Anspruch 1, welches an einen festen Träger gebunden ist.

**4.** Mycoplasma-Antigen nach Anspruch 1, dadurch gekennzeichnet, daß das Mycoplasma ausgewählt ist aus der Gruppe aus M. pneumoniae FH, M. pneumoniae PI 1428, M. hominis, M. fermentans, Ureaplasma urealyticum, M. orale, M. pulmonis, M. salivarium and Acholeoplasma laidlawii.

**5.** M. pneumonia-Antigen nach Anspruch 4, dadurch gekennzeichnet, daß die Proteine desselben ein Molekulargewicht im Bereich von 10.000 bis etwa 60.000 Daltons besitzen.

**6.** Verfahren zur Herstellung eines Mycoplasma-Antigens nach einem der Anspruch 1 bis 5, gekennzeichnet durch die Schritte des Lysierens eines Mycoplasmas und des Unterwerfens der Membran derselben unter DNAse- und RNAse-Verdauung und Beschallung.

**7.** Verfahren nach Anspruch 6, dadurch gekennzeichnet, daß besagte Verdauung der Beschallung vorangeht.

**8.** Verfahren nach Anspruch 6 oder 7, dadurch gekennzeichnet, daß die DNAse-Menge von etwa 100 bis etwa 500 K-Einheiten pro 1 mg Mycoplasma-Membranprotein beträgt.

9. Verfahren nach einem der Ansprüche 6 bis 8, dadurch gekennzeichnet, daß die RNAse-Menge von etwa 10 bis etwa 50 K-Einheiten pro 1 mg Mycoplasma-Membranprotein beträgt.

10. Verfahren nach einem der Ansprüche 6 bis 9, dadurch gekennzeichnet, daß die Beschallung intermittierend ist, wobei jede einzelne Beschallungsperiode etwa 40 bis etwa 90 Sekunden dauert.

11. Mycoplasma-Antigen nach einem der Ansprüche 1 bis 5, dadurch gekennzeichnet, daß es nach dem Verfahren nach einem der Ansprüche 6 bis 10 hergestellt ist.

12. Konjugat, gekennzeichnet durch ein Mycoplasma-Antigen nach einem der Ansprüche 1 bis 5 und 11, welches an einen Marker gebunden ist.

13. Konjugat nach Anspruch 12, dadurch gekennzeichnet, daß die Marker-Einheit ein Enzym ist.

14. Konjugat nach Anspruch 13, dadurch gekennzeichnet, daß das Enzym ausgewählt ist aus der Gruppe aus alkalischer Phosphatase, Meerrettich-Peroxidase, -Galactosidase, Glucose-6-phosphat-Dehydrogenase, Glucose-Oxidase, Lysosym, Glucoamylase und Maleat-Dehydrogenase.

15. Konjugat nach Anspruch 11, dadurch gekennzeichnet, daß der Marker ausgewählt ist aus der Gruppe aus radioaktiven Substanzen, die in der Lage sind, sich mit Mycoplasma-Antigen zu konjugieren, fluoreszierenden Farbstoffen, Biotin-Avidin-Komplexen und chemilumineszenten Substanzen, die alle konventionell als Marker verwendet werden.

16. Konjugat mit alkalischer Phosphatase nach Anspruch 14, dadurch gekennzeichnet, daß es aus einer Ausgangssuspension hergestellt ist, die etwa 800-1200 Einheiten alkalische Phosphatase zu 1 mg Mycoplasma-Membranproteinen umfaßt.

17. In-vitro-Verfahren zur Diagnose von Mycoplasma-Infektionen in einem Menschen oder einem nichtmenschlichen Tier, welches den in-vitro-Nachweis von Antikörpern gegen Mycoplasma in einer Körperflüssigkeit umfaßt, gekennzeichnet durch die Verwendung eines Mycoplasma-Antigens nach einem der Ansprüche 1 bis 5 und 11.

18. In-vitro-Verfahren zur Diagnose von Mycoplasma-Infektionen in einem Menschen oder einem nichtmenschlichen Tier, welches den in-vitro-Nachweis von Antikörpern gegen Mycoplasma in einer Körperflüssigkeit umfaßt, gekennzeichnet durch die Verwendung eines Mycoplasma-Antigen-Konjugats nach einem der Ansprüche 12 bis 16.

19. Verfahren nach Anspruch 18, gekennzeichnet durch:
    (a) Inkubieren einer nativen oder verdünnten Körperflüssigkeitsprobe mit einer Matrix, die wenigstens eine Art von Anti-Isotyp-Antikörpern trägt, ausgewählt aus der Gruppe aus Anti-IgM, Anti-IgG, Anti-IgA, Anti-IgD und Anti-IgE;
    (b) Entfernen der Flüssigkeitsprobe von der Matrix und Waschen der Matrix;
    (c) Inkubieren der Matrix mit einer wäßrigen Suspension eines Konjugats nach einem der Ansprüche 13 bis 18; und
    (d) Bestimmen der Menge von besagten Konjugat auf der Matrix.

20. Verfahren nach Anspruch 19, gekennzeichnet durch die Verwendung eines Konjugats, bei dem die Marker-Einheit ein Enzym und die Bestimmung der Menge von besagten Konjugat auf der Matrix umfaßt:
    (a) Inkubation in einem wäßrigen Substratmedium für das Enzym im Konjugat;
    (b) Bestimmung der Konzentration des Substrats oder eines Abbauproduktes desselben.

21. Für die Durchführung des Verfahrens der Ansprüche 19 oder 20, ein Kit, gekennzeichnet durch:
    (a) eine Matrix, die wenigstens einen Typ von Anti-Isotyp-Antikörpern trägt, ausgewählt aus der Gruppe, die aus Anti-IgM, Anti-IgG, Anti-IgE, Anti-IgD und Anti-IgA besteht; und
    (b) eine Menge eines Konjugats nach einem der Ansprüche 12 bis 16.

**22.** Kit nach Anspruch 21, in dem besagtes Konjugat eine Enzym-Marker-Einheit besitzt, gekennzeichnet durch Einbeziehen eines Substrats für eine enzymatische Reaktion.

**Revendications**

**1.** Matière de membrane de mycoplasme fragmentée, appelée ici antigène de mycoplasme, caractérisée en ce qu'elle est essentiellement exempte d'ADN et d'ARN.

**2.** Antigène de mycoplasme selon la revendication 1, en suspension dans une solution aqueuse.

**3.** Antigène de mycoplasme selon la revendication 1, lié à un support solide.

**4.** Antigène de mycoplasme selon la revendication 1, caractérisé en ce que le mycoplasme est choisi dans le groupe constitué par M. pneumoniae FH, M. pneumoniae PI 1428, M. hominis, M. fermentans, Ureaplasma urealyticum, M. orale, M. pulmonis, M. salivarium et Acholeoplasma laidlawii.

**5.** Antigène de M. pneumonia selon la revendication 4, caractérisé en ce que les protéines de celui-ci ont un poids moléculaire dans la plage d'environ 10000 à environ 60000 Daltons.

**6.** Procédé de préparation d'un antigène de mycoplasme selon l'une quelconque des revendications 1 à 5, caractérisé par les étapes consistant à faire une lyse d'un mycoplasme et à soumettre la membrane de celui-ci à une digestion par désoxyribonucléase et ribonucléase et à un traitement aux ultrasons.

**7.** Procédé selon la revendication 6, caractérisé en ce que ladite digestion précède le traitement aux ultrasons.

**8.** Procédé selon la revendication 6 ou 7, caractérisé en ce que la quantité de désoxyribonucléase est d'environ 100 à environ 500 K unités par mg de protéine de membrane de mycoplasme.

**9.** Procédé selon l'une quelconque des revendications 6 à 8, caractérisé en ce que la quantité de ribonucléase est d'environ 10 à environ 50 K unités par mg de protéine de membrane de mycoplasme.

**10.** Procédé selon l'une quelconque des revendications 6 à 9, caractérisé en ce que le traitement aux ultrasons est intermittent, chaque période individuelle de traitement aux ultrasons durant environ 40 à environ 90 secondes.

**11.** Antigène de mycoplasme selon l'une quelconque des revendications 1 à 5, caractérisé en ce qu'il est préparé à l'aide du procédé selon l'une quelconque des revendications 6 à 10.

**12.** Conjugué caractérisé par un antigène de mycoplasme selon l'une quelconque des revendications 1 à 5 et 11 lié à un marqueur.

**13.** Conjugué selon la revendication 12, caractérisé en ce que la fraction formant le marqueur est une enzyme.

**14.** Conjugué selon la revendication 13, caractérisé en ce que l'enzyme est choisie dans le groupe constitué par la phosphatase alcaline, la peroxydase de raifort, la -galactosidase, la glucose-6-phosphate déshydrogénase, la glucose oxydase, la lysozyme, la glucoamylase et la maléate déshydrogénase.

**15.** Conjugué selon la revendication 11, caractérisé en ce que le marqueur est choisi dans le groupe constitué par les substances radio-actives capables de se conjuguer avec l'antigène de mycoplasme, les colorants fluorescents, les complexes biotine-avidine et les substances chimiluminescentes qui sont toutes utilisées de manière classique comme marqueurs.

**16.** Conjugué de phosphatase alcaline selon la revendication 14, caractérisé en ce qu'il est préparé à partir d'une suspension de départ comprenant environ 800 à 1200 unités de phosphatase alcaline par mg de protéines de membrane de mycoplasme.

**17.** Procédé in-vitro de diagnostic d'infection mycoplasmique chez un être humain ou un animal, comportant la détection in-vitro d'anti-corps du mycoplasme dans un fluide corporel, caractérisé par l'utilisation d'un antigène de mycoplasme selon l'une quelconque des revendications 1 à 5 et 11.

**18.** Procédé in-vitro de diagnostic d'infection mycoplasmique chez l'être humain ou un animal, comportant la détection in-vitro d'anti-corps du mycoplasme dans un fluide corporel, caractérisé par l'utilisation d'un conjugué d'antigène de mycoplasme selon l'une quelconque des revendications 12 à 16.

**19.** Procédé selon la revendication 18, caractérisé par les opérations suivantes :
(a) on procède à l'incubation d'un échantillon de fluide corporel natif ou dilué avec une matrice comportant au moins un type d'anti-corps anti-isotype choisi parmi le groupe constitué par l'anti-IgM, l'anti-IgG, l'anti-IgA, l'anti-IgD et l'anti-IgE;
(b) on retire de la matrice l'échantillon de fluide et on lave la matrice ;
(c) on procède à l'incubation de la matrice avec une suspension aqueuse d'un conjugué selon l'une quelconque des revendications 13 à 18; et
(d) on détermine la quantité dudit conjugué sur la matrice.

**20.** Procédé selon la revendication 19, caractérisé par l'utilisation d'un conjugué dans lequel la fraction formant le marqueur est une enzyme et en ce que la détermination de la quantité dudit conjugué sur la matrice comprend les opérations suivantes :
(a) on procède à l'incubation avec un milieu aqueux formant substrat pour l'enzyme dans le conjugué;
(b) on détermine la concentration du substrat ou d'un produit de dégradation de celui-ci.

**21.** Pour la mise en oeuvre du procédé des revendications 19 ou 20, ensemble caractérisé par :
(a) une matrice portant au moins un type d'anti-corps anti-isotype choisi dans le groupe constitué par l'anti-IgM, l'anti-IgG, l'anti-IgE, l'anti-IgD et l'anti-IgA; et
(b) une quantité d'un conjugué selon l'une quelconque des revendications 12 à 16.

**22.** Ensemble selon la revendication 21, dans lequel ledit conjugué possède une fraction formant le marqueur qui est une enzyme, caractérisé en ce qu'il comprend un substrat pour une réaction enzymatique.

FIG. 1

FIG. 2